(19) Europäisches Patentamt
European Patent Office
Office européen des brevets

(11) Publication number: **0 440 298 A1**

# (12) EUROPEAN PATENT APPLICATION

(21) Application number: **91200166.6**

(51) Int. Cl.5: **A61K 7/04**, A61K 31/195

(22) Date of filing: **28.01.91**

(30) Priority: **30.01.90 EP 90200209**

(43) Date of publication of application: **07.08.91 Bulletin 91/32**

(84) Designated Contracting States: **AT BE CH DE DK ES FR GB GR IT LI LU NL SE**

(71) Applicant: **GIST-BROCADES N.V.**
**Wateringseweg 1 P.O. Box 1**
**NL-2600 MA Delft(NL)**

(72) Inventor: **van den Hoven, Wilhelm Elbertus**
**Kalmoes 2**
**NL-2771 LG Boskoop(NL)**
Inventor: **Olthoff, Margaretha**
**Hammerskjoldlaan 129**
**NL-2286 HA Rijswijk(NL)**

(74) Representative: **Lavy, Uriel et al**
**Gist-Brocades NV Patents and Trademarks Dept. Wateringseweg 1 P.O. Box 1**
**NL-2600 MA Delft(NL)**

## (54) Topical preparations for treating human nails.

(57) The use of sulphur containing amino acids as represented by the general formula

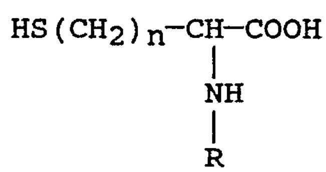

wherein

n = 1-4

R = H, alkyl having 1-12 C atoms, or carboxylic acyl, or pharmaceutically acceptable salts thereof,

in particular N-acetyl-cysteine (NAC), for the preparation of topical compositions for the medical or cosmetic treatment of human nails. The topical compositions containing these compounds may be applied to the nails separately from or in combination with topically effective medicaments.

EP 0 440 298 A1

# TOPICAL PREPARATIONS FOR TREATING HUMAN NAILS

## Background of the invention

This invention relates to topical preparations for the medical or cosmetic treatment of human nails.

The permeability, absorption and retention capacity of the human nail plate pose a clinical problem in the topical treatment of various nail diseases such as onychomycosis, psoriasis and lichen planus.

It is well known, e.g. from K.A. Walters and G.L. Flynn, Internat. J. Cosmet. Sci. 5, 1983, 231-246, that the nail plate is comprised of flattened keratinized cells fused into a dense, but somewhat elastic mass. Its amino acid composition is qualitatively similar to that of the stratum corneum and the hair, but quantitatively it is more like hair than like stratum corneum and this is also true of its thermoanalytical, mechanical and moisture-retaining properties. While the permeability pattern of skin shows it to function to a great extent as a lipid barrier, the nail plate appears to exclude compounds as they become more hydrophobic, which is an exactly opposite behaviour. The water-retaining capacity of nails is normally about 20%, much less than that of stratum corneum. Solvents with proven efficacy as skin penetration enhancers, such as dimethyl sulphoxide (DMSO) and isopropanol, do not have the same effect on the nail plate. The nail plate thus has proved to be a unique structure, calling for specific methods of treatment.

In the treatment of the above-mentioned nail diseases, systemic (oral) medication is mostly used. Especially in the case of onychomycosis, probably due to the relative inaccessibility of the "dead" nail plate to systemic medication, such treatment generally requires many months, with often disappointing results. Besides, it may involve undesirable side-effects.

Another possibility of treating nail diseases is to remove the nails surgically or chemically and then apply the medicament locally to the nail beds. The chemical avulsion has been effected with 22% to 40% urea on dystrophic nails (E.M. Farber and D.A. South, Cutis 22, 1978, 689-692) and with a combination of 20% urea and 10% salicylic acid on nondystrophic nails (T.J. Buselmeier, Cutis 25, 1980, 297-405). D. Kulenkamp reports in Der Deutsche Dermatologe 36, 1988, 3-7 the treatment of onychomycosis by nail-avulsion with 40% urea, followed by local treatment of the nail bed with 1% bifonazol. All the above-described local treatments involve the loss of the diseased nails, whereby the occurrence of infection and other complications may be increased.

There thus exists a need for a conservative and rapidly effective method of locally treating diseased nails, without having to remove them. Topical preparations which could carry therapeutic agents into and through the nail plate, also enhancing the capacity of the nail plate to retain the medicaments, would fill this need.

N.F. Wolejza et al., in J. Soc. Cosmet. Chem. 22, 1971, 571-578, propose the partial or complete reduction of structural cysteine in fingernail keratin, in order to enhance the internal diffusibility and subsequent binding of water and other materials. Said publication contains no recommendation as to the utility of this effect. The reducing reagents tested were ammonium thioglycolate, ammonium bisulphite, 1,4-dithiothreitol and tetrakis(hydroxymethyl) phosphonium chloride. None of those were ever actually used in topical preparations for the medical or cosmetic treatment of human nails, before or after the date of said publication (1971).

## Summary of the invention

The present invention is directed to the use of sulphur containing amino acids represented by the general formula

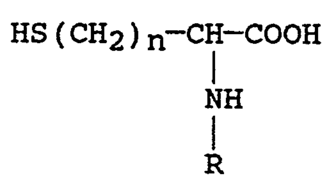

wherein

n = 1-4

R = H, alkyl having 1-12, preferably 1-4 C atoms, or carboxylic acyl, or pharmaceutically acceptable

salts thereof,

for the preparation of topical compositions for the medical or cosmetic treatment of human nails.

In particular, the invention is related to such use of compounds according to the above formula, wherein the acyl is an acyl of an aliphatic mono- or dicarboxylic acid of 1-12 carbon atoms.

## Further details of the invention

Examples of compounds according to the invention are N-acetyl-cysteine, N-acetyl-homo-cysteine, N-ethyl-cysteine and N-malyl-cysteine.

Such compounds are known to reduce S-S bonds and several of them are in use for hair treatment (permanent waving) and also in medicines not related to nail treatment. They have now been found to have the useful property of softening and hydrating the nail plate, thereby increasing its permeability and absorption capacity for both lipophilic and hydrophilic substances, in particular medicaments and dyes.

Suitable concentrations of the above compounds in the topical compositions according to the invention are 1-40% w/v, preferably 5-40% w/v, more preferably 10-20% w/v.

The compounds according to the invention can be formulated into any type of topical formulation which is suitable for application to the nail plate. Examples of such formulations are ointments, lotions, film-forming lotions, pastes, creams and gels, including instant preparations. These may be prepared according to methods known in the art.

Preferably, the compounds according to the invention are N-carboxylic acyl substituted derivatives of cysteine.

The most preferred compound according to the present invention is N-acetyl-cysteine (NAC). NAC is currently used as a mucolytic in treating adverse conditions of the respiratory tract. NAC has also been incorporated in preparations for the treatment of the skin, as in European Patent Application 0300100, which is directed to the treatment of certain dermal inflammations. By contrast to urea, salicylic acid and their combinations, NAC does not cause the maceration of the nail plate. NAC is neither toxic nor allergenic.

Suitable NAC concentrations in the above topical formulations are 1-40% w/v, preferably 5-40% w/v, more preferably 10-20% w/v.

The topical formulations containing NAC should also contain a certain amount of water, preferably between 5% and 40% v/v, more preferably between 5% and 20% v/v. The pH should be less than 5, preferably between 1-3.

Therapeutic agents or dyes of which the penetration through and absorption into the nail plate is to be enhanced by the sulphur containing amino acids according to the invention, may be formulated together with these compounds. Alternatively the therapeutic agents or dyes can be formulated separately, to be applied to the nail plate after a pretreatment with a topical formulation containing the sulphur containing amino acids.

Examples of therapeutic agents for the treatment of the human nail which can suitably be combined with the sulphur containing amino acids according to the invention are antimycotic medicaments such as imidazoles, in particular oxiconazole and miconazole, allylamines and butylamines, in particular N-(4-tert-butylbenzyl),N-methyl-1-naphtylmethylamine (butenafine, KP-363), anti-inflammatory and/or antiproliferative and antitumor compounds such as corticosteroids, in particular hydrocortisone 17-butyrate, betamethason 17-valerate, budesonide and clobetasol 17-propionate, retinoids, 5-fluorouracil, podophylotoxin and bleomycin, antibacterials like pseudomonic acid and bacitracin, and antivirus compounds such as $\alpha$-interferon.

Another application of the sulphur containing amino acids according to the invention is to combine them with dyes for the cosmetic treatment of normal and of discoloured nails.

The following Examples illustrate the invention.

## Example 1

### Weight increase of nail clippings during immersion in a lotion

Solutions (lotions) were prepared, containing (v/v) 40% of distilled water, 40% of methyl ethyl ketone and 20% of isopropyl alcohol.

To these lotions were added salicylic acid, NAC, 1,4-dithiothreitol or mercaptoethylamine, in w/v concentrations as further specified in Table 1.

To distilled water were added glutathione, mercaptoethanol or urea, in w/v concentrations as further specified in Table 1.

Human nail clippings were immersed in the test and control lotions at ambient temperature and their weight increase expressed as % of their original weight. The results are presented in the following Table 1:

Table 1

| Substance tested | Concentration % w/v | Nail clippings | | | | | | |
|---|---|---|---|---|---|---|---|---|
| | | n* | Average % weight increase during immersion (hours) | | | | | |
| | | | 1 | 3 | 6 | 24 | 48 | 72 |
| Distilled water | - | 8 | 20 | 19 | 24 | 22 | 22 | 24 |
| Blanc lotion | - | 6 | 12 | 17 | 19 | 21 | 23 | 24 |
| Salicylic acid | 14 | 2 | 17 | 24 | 26 | 29 | 30 | 30 |
| NAC | 5 | 3 | 23 | 32 | 38 | 47 | 60 | 70 |
| " | 10 | 3 | 24 | 33 | 38 | 59 | 67 | 79 |
| " | 20 | 3 | 23 | 30 | 37 | 60 | 91 | 110 |
| " | 40 | 3 | 24 | 33 | 38 | 71 | 110 | 154 |
| 1,4-Dithiothreitol | 20 | 2 | 21 | 32 | 38 | 51 | 50 | 58 |
| Mercaptoethyl-amine | 20 | 2 | 10 | 16 | 21 | 38 | 55 | 64 |
| Glutathione | 10 | 2 | 30 | 32 | 33 | 44 | 58 | 61 |
| Urea | 50 | 2 | 22 | 24 | 32 | 35 | 36 | 37 |

* = number of clippings

## Example 2

### Penetration of the hydrophilic dye eosine in nail clippings during immersion in a lotion

To a similar solution (lotion) as in Example 1, consisting of (v/v) 40% distilled water, 40% of methyl ethyl ketone and 20% of isopropyl alcohol, were added (w/v) 17% of NAC and 1% of eosine.

Human nail clippings were immersed in this liquid and in the same liquid without NAC (n = 2 x 3) during 48 hours at room temperature, after which the colour penetration was estimated on microscopic freeze sections.

The average penetration of the dye in the NAC-treated nails was 27.5% of the total nail thickness, compared to an average of 6% in the controls.

## Example 3

### Penetration of the hydrophilic dye eosine in nail clippings after immersion in a lotion

The same experimental set-up as in Example 2 was used, except that the nail clippings were first immersed in lotions lacking eosine, and subsequently during 24 hours in lotions containing 1% eosine but lacking NAC. n was again 2 x 3.

The average penetration of the dye through the NAC-pretreated nails was now 19%, compared to an

average of 4% in the controls.

**Example 4**

**Weight increase and penetration of eosine in nail clippings as a result of dorsal application of a film-forming lotion**

A film-forming lotion was prepared, consisting by weight of 17.5 parts of distilled water, 17.5 parts of ethanol, 20 parts of acetone, 25 parts of polyvinylpyrrolidone VA-S630 and 20 parts of NAC. The control film-forming lotion lacked the NAC.

Human nail clippings (n = 2 x 3) were laid on pads soaked in saline, and the two types of film-forming lotion were painted on their dorsal surface twice a day. The weights of the clippings were determined after 3, 4, 5 and 6 days.

The weight of the NAC-treated clippings increased by 34% after 3 days and did not change further. The weight of the controls was increased by 14% after 3 days and also did not change further.

After the above 6 days of treatment, the (2 x 3) clippings were rinsed and placed for 24 hours in a solution of 1% eosine, as described in Example 2 (without NAC), and then further processed for the microscopic estimation of dye penetration as in Example 2. The average penetration of the dye through the NAC-penetrated nails was 48% of the nail thickness, compared to 6% in the controls.

**Example 5**

**Weight increase and penetration of eosine in nail clippings as a result of treatment with a cream**

A cream base was prepared, consisting of (weight parts):

| | |
|---|---|
| white paraffin | 150 |
| liquid paraffin | 60 |
| cetostearyl alcohol | 72 |
| Cetomacrogol 1000® | 18 |
| Nipagin M® | 1.5 |
| distilled water | 618.5 |

To 88.8 grams of this cream base were added 10 grams of NAC, 1.1 grams of disodium phosphate (12 aq.) and 0.1 gram of sodium disulphite to make a NAC cream of pH 2.

To 98 grams of the above cream base were added 1.02 grams of phosphoric acid 85%, 0.85 grams of disodium phosphate and 0.1 gram of sodium disulphite to make a control cream of pH 2.

The two sorts of cream were used for either immersion (as in Example 1) or dorsal painting twice daily (as in Example 4) of human nail clippings for a total period of 5 days (n = 4 x 3). Their weight increase was determined daily, and at the end of 5 days the clippings were rinsed and their penetration of eosine was determined as in Example 4.

The results are summarized in the following Table 2:

Table 2

| Substance tested | Treatment | Nail clippings | | | | |
|---|---|---|---|---|---|---|
| | | n | Average % weight increase after treatment during (days) | | | Average % penetration of eosine |
| | | | 1 | 2 | 5 | |
| NAC cream | immersion | 3 | 34 | 45 | 68 | 60 |
| Control cream | immersion | 3 | 27 | 26 | 32 | 8 |
| NAC cream | dorsal painting | 3 | 22 | 25 | 45 | 38 |
| Control cream | dorsal painting | 3 | 19 | 22 | 25 | 9 |

**Example 6**

**Effect of treatment with NAC-containing lotion on the penetration of oxiconazole in human nail discs**

A 20% w/v NAC-containing lotion and a control lotion were prepared as in Example 1.

To both these preparations was added 1% w/v of oxiconazole, containing a known amount (1 µCi per 30 µl) of radioactive $^{14}C$ oxiconazole.

On human nail discs of 6 mm diameter were mounted 1.5 cm pieces of tubing of 4 mm outer and 3 mm inner diameter, in each of which was poured 30 µl of one of the above lotions (n = 2 x 3). The free (ventral) surfaces of the nail discs were kept moist on a pad soaked in saline.

After 48 hours of incubation at 30°C and the discs were sectioned (50 µm) parallel to their dorsal surface, using a freeze-microtome. Radioactivity was measured in the separate sections, after which the amount of penetrated oxiconazole was calculated.

The average total uptake of oxiconazole in the NAC-treated discs (expressed as a % of the total amount of oxiconazole applied) was 4.94%, compared to 1.33% in the control-treated discs.

Table 3 shows that in the NAC-treated discs, in contrast to control treatment, substantial amounts of oxiconazole could be detected up into the deeper layers.

Table 3

| Substance tested | n | Average amount of oxiconazole (µg) per nail disc section of depth (µm) | | | |
|---|---|---|---|---|---|
| | | 100-150 | 200-250 | 400-450 | 600-650 |
| NAC lotion | 3 | 2.05 | 0.46 | 0.26 | 0.14 |
| Control lotion | 3 | 0.45 | 0.05 | 0.02 | 0.01 |

## Example 7

**Effect of pretreatment with NAC-containing film-forming lotion on the penetration of oxiconazole in the human nail discs**

A 20% w/v NAC-containing film-forming lotion and a control film-forming lotion were prepared as in Example 4.

Human nail discs of 6 mm diameter, resting with their ventral side on pads soaked with saline, were dorsally painted with these film-forming lotions (n = 2 x 3) twice daily during 5 days. Then the six nail discs were rinsed and mounted on pieces of tubing as in Example 6. Subsequently 20 mg of $^{14}C$ oxiconazole was added to 30 ml of Oceral® lotion (i.e. 1% oxiconazole in an alcoholic base). Of this, 30 $\mu$l was poured into the tubings on top of the dorsal surfaces of the discs as in Example 6. The free (ventral) surfaces of the nail discs were again kept moist on a pad soaked in saline.

After 48 hours of incubation at 30°C and 60% relative humidity the discs were sectioned (50 $\mu$m) parallel to their dorsal surface, using a freeze-microtome. Radioactivity was measured in the separate sections, after which the amount of penetrated oxiconazole was calculated.

The average total uptake of oxiconazole in the NAC-pretreated discs (expressed as a % of the total amount of oxiconazole applied) was 3.25%, compared to 0.29% in the control-pretreated discs.

The average radioactivity in the three pads under the NAC-pretreated discs was 7.53%, as compared to 0.01% radioactivity in the three pads under the control-pretreated discs.

Table 4 shows that NAC pretreatment, in contrast to control pretreatment, resulted in higher amounts of oxiconazole, even in the deeper layers of the discs.

**Table 4**

| Substance tested | n | Average amount of oxiconazole ($\mu$g) per nail disc section of depth ($\mu$m) | | | |
|---|---|---|---|---|---|
| | | 100-150 | 200-250 | 400-450 | 600-650 |
| NAC film-forming lotion | 3 | 1.48 | 1.08 | 0.20 | 0.11 |
| Control film-forming lotion | 3 | 0.11 | 0.02 | 0.02 | 0.01 |

## Example 8

**Effect of pretreatment with cream on the penetration of oxiconazole in the human nail disc**

A NAC-containing cream and a control cream were prepared as in Example 5.

Human nail discs of 6 mm diameter, resting with their ventral side on pads soaked with saline, were dorsally painted with these creams (n = 2 x 3) twice daily during 5 days. Then the six nail discs were rinsed and mounted on pieces of tubing as in Example 6. Subsequently 20 mg of $^{14}C$ oxiconazole was added to 30 ml of Oceral® lotion (i.e. 1% oxiconazole in an alcoholic base). Of this, 30 $\mu$l was poured into the tubings on top of the dorsal surfaces of the discs as in Example 6. The free (ventral) surfaces of the nail discs were again kept moist on a pad soaked in saline.

After 48 hours of incubation at 30°C and 60% relative humidity the discs were sectioned (50 $\mu$m) parallel to their dorsal surface, using a freeze-microtome. Radioactivity was measured in the separate sections, after which the amount of penetrated oxiconazole was calculated.

The average total uptake of oxiconazole in the NAC-pretreated discs (expressed as a % of the total amount of oxiconazole applied) was 4.0%, compared to 0.22% in the control-pretreated discs.

Table 5 shows the amount of oxiconazole in the separate sections revealing that NAC-pretreatment, in contrast to control pretreatment, resulted in the presence of high amounts of oxiconazole, even in the deeper layers.

Table 5

| Substance tested | n | Average amount of oxiconazole (μg) per nail disc section of depth (μm) | | | |
|---|---|---|---|---|---|
| | | 100-150 | 200-250 | 400-450 | 600-650 |
| NAC cream | 3 | 1.74 | 1.46 | 0.74 | 0.25 |
| Control cream | 3 | 0.10 | 0.06 | 0.03 | 0.01 |

From the combined results of Examples 6, 7 and 8 it may be concluded that NAC promotes the penetration of the lipophilic therapeutic agent oxiconazole both in and through the nail plate.

**Example 9**

**Effect of treatment with NAC-containing lotion on the penetration of hydrocortisone in human nail discs**

The experimental set-up of Example 6 was repeated, with a 20% w/v NAC-containing lotion and a control lotion, both prepared as in Example 1 and both containing 1% w/v of hydrocortisone, wherein a known amount (1 μCi per 30 μl) of radioactive $^{14}C$ hydrocortisone.

Table 6 shows that in the NAC-treated discs substantially more hydrocortisone could be detected than in the controls, especially in the deeper layers.

Table 6

| Nail disc section (μm) | n | Average amount of hydrocortisone (μg) | |
|---|---|---|---|
| | | NAC lotion | Control lotion |
| 0- 50 | 3 | 0.99 | 0.27 |
| 50-100 | 3 | 1.08 | 0.75 |
| 100-150 | 3 | 1.15 | 0.77 |
| 150-200 | 3 | 1.23 | 0.57 |
| 200-250 | 3 | 1.44 | 0.39 |
| 250-300 | 3 | 1.34 | 0.28 |
| 300-350 | 3 | 1.12 | 0.13 |
| 350-400 | 3 | 1.05 | 0.12 |
| 400-450 | 3 | 0.91 | 0.04 |
| 450-500 | 3 | 0.76 | 0.02 |
| 500-550 | 3 | 0.63 | 0.02 |
| 550-600 | 3 | 0.45 | 0.02 |
| 600-650 | 3 | 0.30 | 0.02 |
| 650-700 | 3 | 0.25 | 0.02 |
| 700-750 | 3 | 0.16 | 0.05 |
| 750-800 | 3 | 0.12 | 0.06 |
| Total | | 12.98 | 3.53 |

## Example 10

### Comparison of the effect of different sulfhydrdryl compounds on the weight increase of nail clippings during immersion in a lotion

The effects of 0.1 M of each of NAC (pH 2.3), 1,4-dithiothreitol (DTT, not claimed, pH 4.5) and ammonium thioglycolate (ATG, not claimed, pH 9.0), in a lotion consisting of 50% v/v ethanol in distilled water were compared, as in Example 1. The immersion period of the human nail clippings in the control and test lotions was 72 hours. The results are presented in the following Table 7.

Table 7

| Substance tested | Concentration | | n | Average % weight increase after immersion during 72 hours |
| --- | --- | --- | --- | --- |
| | % w/v | M | | |
| Blank lotion | - | - | 3 | 19 |
| NAC | 1.63 | 0.1 | 3 | 72 |
| 1,4-Dithiothreitol | 1.54 | 0.1 | 3 | 49 |
| Ammonium thioglycolate | 1.08 | 0.1 | 3 | 52 |

## Example 11

### Comparison of the effect of three different sulfhydryl compounds in a lotion on the penetration of oxiconazole in human nail discs

To lotions consisting of 50% v/v of ethanol in distilled water were added 0.1 M of NAC (pH 2.3), of 1,4-dithiothreitol (DTT, not claimed, pH 4.5) or of ammonium thioglycolate (ATG, not claimed, pH 9.0), as in Example 10. To all three solutions was also added 1% w/v of oxiconazole, containing a known amount (1 $\mu$Ci per 30 $\mu$l) of radioactive $^{14}$C oxiconazole. These lotions were tested in an experimental set-up as in Examples 6 and 9.

Table 8 shows that in the NAC-treated discs substantially more oxiconazole could be detected than in the other two groups of discs, especially in the deeper layers.

Table 8

| Nail disc section (μm) | n | Average amount of oxiconazole (μg) | | |
|---|---|---|---|---|
| | | NAC | DTT | ATG |
| 0- 50 | 2 | 3.06 | 3.10 | 0.96 |
| 50-100 | 2 | 2.99 | 3.40 | 0.60 |
| 100-150 | 2 | 2.48 | 1.40 | 0.43 |
| 150-200 | 2 | 1.53 | 0.90 | 0.16 |
| 200-250 | 2 | 1.51 | 0.70 | 0.05 |
| 250-300 | 2 | 1.18 | 0.17 | 0.05 |
| 300-350 | 2 | 1.08 | 0.10 | 0.02 |
| 350-400 | 2 | 0.46 | 0.10 | 0.02 |
| 400-450 | 2 | 0.44 | 0.10 | 0.01 |
| 450-500 | 2 | 0.26 | 0.01 | 0.01 |
| 500-550 | 2 | 0.13 | 0.01 | 0.01 |
| 550-600 | 2 | 0.02 | 0.01 | 0.01 |
| 600-650 | 2 | 0.01 | 0.01 | 0.01 |
| 650-700 | 2 | 0.01 | 0.01 | 0.00 |
| 700-750 | 2 | 0.01 | 0.00 | 0.00 |
| 750-800 | 2 | 0.00 | 0.00 | 0.00 |
| Total | | 15.17 | 10.02 | 2.34 |

## Claims

1. The use of a sulphur containing amino acid represented by the general formula

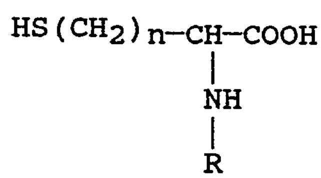

wherein
n = 1-4
R = H, alkyl having 1-12 C atoms, or carboxylic acyl, or a pharmaceutically acceptable salt thereof,
for the preparation of topical compositions for the medical or cosmetic treatment of human nails.

2. Use according to claim 1, characterized in that the sulphur containing amino acid is a N-carboxylic acyl

substituted derivative of cysteine.

3. Use according to claim 2, characterized in that the cysteine derivative is N-acetyl-cysteine (NAC).

4. Use according to claim 3, characterized in that the concentration of NAC in the topical composition is between 1-40% w/v, preferably between 5-40% w/v, more preferably between 10-20% w/v.

5. Use according to any one of claims 1-4, characterized in that the topical composition contains a topically effective therapeutic agent.

6. Use according to claim 5, characterized in that the therapeutic agent is an imidazole compound.

7. Use according to claim 6, characterized in that the imidazole compound is oxiconazole.

8. Use according to claim 5, characterized in that the medicament is N-(4-tert-butylbenzyl),N-methyl-1-naphtyl-methylamine (butenafine, KP-363),

9. Use according to claim 5, characterized in that the medicament is a corticosteroid.

10. Use according to claim 9, characterized in that the corticosteroid is budesonide.

11. Use according to any one of claims 1-10, characterized in that the topical composition contains a dye.

12. Use according to any one of claims 1-11, characterized in that the topical composition is a lotion, a film-forming lotion, a cream, an ointment, a gel or a paste, including instant preparations.

13. Topical compositions for the medical or cosmetic treatment of human nails, characterized in that they contain from 1% to 40% w/v of a sulphur containing amino acid respresented by the general formula

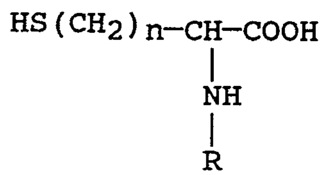

wherein

n = 1-4

R = H, alkyl having 1-12 C atoms, or carboxylic acyl, or a pharmaceutically acceptable salt thereof, and a pharmaceutically acceptable carrier.

European Patent Office

# EUROPEAN SEARCH REPORT

Application Number

**EP 91 20 0166**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X<br><br>Y | DE-B-2 840 498 (SCHWECKENDIEK)<br>* Whole document * | 1,5,12-13<br><br>1-5,12-13 | A 61 K 7/04<br>A 61 K 31/195 |
| Y | FR-A-1 485 602 (MORELLE)<br>* Whole document * | 1-5,12-13 | |
| Y | US-A-4 827 016 (MORGAN)<br>* Whole document * | 1-5,12-13 | |
| X | FR-A-2 503 151 (MORELLE et al.)<br>* Whole document * | 1,12-13 | |
| X | FR-A-2 094 109 (LUBOWE)<br>* Whole document * | 1,5-6, 12-13 | |
| X | GB-A-2 165 453 (ROHM GmbH)<br>* Claims; page 2, lines 84-93; page 1, lines 12-17 * | 1,5,13 | |
| X | WO-A-8 907 930 (CIRO'S TOUCH LTD)<br>* Page 8, line 38 - page 9, line 7; page 4, lines 14-23; claims * | 1,5,12-13 | **TECHNICAL FIELDS SEARCHED (Int. Cl.5)** |
| A | EP-A-0 219 455 (RORY LTD)<br>* Claims * | 1-4,12-13 | A 61 K |

The present search report has been drawn up for all claims

| Place of search | Date of completion of search | Examiner |
|---|---|---|
| The Hague | 03 May 91 | FISCHER J.P. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document